# EUROPEAN PATENT APPLICATION

(11) **EP 4 122 324 A1**
(43) Date of publication of application: **25.01.2023**
(21) Application number: 21382668.8
(22) Date of filing: 22.07.2021
(51) Int. Cl.: A23J 1/10, A23J 3/04, A23K 10/26, A61K 8/65, C12N 1/20

(54) **PROCESS FOR KERATIN CONVERSION**

(71) Applicant: Symborg, S.L., 30100 Murcia (ES)
(72) Inventor: JUAREZ MOLINA, Jesús, 30100 MURCIA (ES); FERNANDEZ MARTIN, Félix, 30100 MURCIA (ES); PARRADO RUBIO, Juan, 30100 MURCIA (ES); ORTS GOMEZ, Ángel, 30100 MURCIA (ES); MARTINEZ RUIZ, Jesús, 30100 MURCIA (ES); CARMONA ALVAREZ, Francisco José, 30100 MURCIA (ES)
(74) Representative: Trupiano, Federica

(57) **Abstract**

The present invention relates to a process for converting keratin into a liquid mixture comprising peptides and/or amino acids comprising the following steps: a) decomposing a keratin containing material, preferably pig hair, in presence of at least one microorganism apt to decompose keratin, to obtain a decomposed keratin containing material; b) treating the decomposed keratin containing material to obtain a modified keratin containing material; c) treating said modified keratin containing material with at least a proteolytic enzyme to obtain a keratin hydrolysate; and d) treating said keratin hydrolysate to obtain a liquid mixture comprising peptides and/or amino acids. The present invention further relates to a liquid mixture obtained from said process and to uses thereof.

## Description

### Field of the invention

The present invention is related to a process of converting keratin into a product, e.g., a mixture including peptides and/or amino acids, suitable to be used in in several technical fields such as, e.g., agriculture, for example, as a bio-stimulant, and animal nutrition.

### Background of the invention

Keratin is considered one of the most abundant animal proteins in nature. Keratin is a fibrous protein and is the major structural component of, for example, animal feathers, hair, hoofs, and nails. For example, in animal hair, the amount of keratin may be even more than 90% by weight. Considering Eurostat data (EU28 countries), about 250 million pigs were slaughtered in the European Union in 2017. Since each slaughtered pig delivers about 0.9 kg of pig bristles (i.e., hair), approximately 225000 tons of wasted pig bristles (i.e., pig hair) are produced annually. Since there are no methods or processes available for the management of such wasted pig bristles, such the discarding for disposal of pig bristles not only results in a waste of protein material, but also causes environmental pollution.

In view of the high keratin content of animal hair (such as pig bristles), as well as birds' feathers, hoofs and nails, such structures contain high amounts of amino acids necessary for nourishment of animals and plants.

However, keratins are hardly digestible by the animals, as well as not absorbable by the plants, because of the highly-structured di-sulphide linked polypeptides which must be cleaved before utilization. Similarly, keratins are not usable, as such, for cosmetic purposes. Degradation of keratin waste can therefore provide an inexpensive source of peptides and amino acids that can be, in turn, employed in multiple uses.

Several methods of converting keratin into peptides and/or amino acids are known.

For example, CN1467179A discloses a high-nitrogen organic fertilizer prepared from keratin through hydrolyzing keratin in alkaline condition, hydrolyzing keratin in acidic condition, mixing the two hydrolysates of keratin and drying. However, the process disclosed in CN1467179A involves the use of hazardous chemicals as well as hazardous chemical reactions.

Therefore, there is a need for processes that allows for the conversion of keratin into more bio-available compounds, such as peptides and/or amino acids, said compounds being in a form that is suitable to be used, for example, in agriculture, e.g., as a biostimulant.

### Summary of the invention

It is an aim of the present invention to solve the above mentioned problems and to provide a process that is effective in converting keratin into more bio-available compounds, such as peptides and/or amino acids.

A further aim of the present invention is to provide a process that is suitable to provide a product, e.g., a mixture comprising peptides and/or amino acids, that can be used as a plant bio-stimulant.

These and other aims are achieved by the present invention, which relates to a process for converting keratin into peptides and/or amino acids, comprising a step of microbial keratin decomposition and a step of enzymatic hydrolysis.

### Detailed description of the invention

Object of the present invention is a process for converting keratin into a liquid mixture comprising peptides and/or amino acids, said process comprising the following steps:
a) decomposing a keratin containing material, preferably pig hair, in presence of at least one species of microorganism apt to decompose keratin, to obtain a partial digested/decomposed keratin containing material;
b) treating the partial digested/decomposed keratin containing material to obtain a modified keratin containing material;
c) treating said modified keratin containing material with at least a proteolytic enzyme to obtain a keratin hydrolysate; and
d) treating said keratin hydrolysate to obtain a liquid mixture comprising peptides and/or amino acids.

According to an aspect, the process of the present invention allows for the conversion of keratin into a hydrolysate, that is further processed to obtain a liquid mixture comprising peptides and/or free amino acids, that are bioactive compounds of high biological bioavailability, for example as plant biostimulants, in cosmetic field or animal nutrition. Advantageously, the liquid mixture obtained according to the process of the invention, is particularly suitable for use in the agricultural field, for example, as agronomic bio-stimulant. In fact, the liquid mixture obtained through the process of the invention is advantageously effective in promoting germination, rooting, growth, flowering, fruit setting and maturation of plants and their fruits. Advantageously, the liquid mixture including peptides and/or amino acids obtained through the process of the invention, can be applied in its different variants to any type of plant, at any stage of plant development, on any soil and form of cultivation, and is also potentially usable in organic farming. For example, in the agriculture field, amino acids/peptides can be applied through different methods such as: foliar application, irrigation (drench, drip irrigation systems, furrows, etc.). They can also be applied in granular form alone or mixed with other fertilizers or biostimulants. Amino acids/peptides can be also applied as seed treatments.

Advantageously, the process of the invention comprises the conversion of keratin-containing materials, e.g., keratin wastes deriving from, for example, animal hair, into a liquid mixture that is suitable to be used as agronomic biostimulant. As above mentioned, the process of the invention includes a step of microbial keratin decomposition, may comprise a step of steam explosion, comprises a step of enzymatic hydrolysis to obtain a keratin hydrolysate, and a step of treating the keratin hydrolysate to obtain a liquid mixture including compounds derived from keratin, such as peptides and/or amino acids, that are more bio-available with respect to keratin. Advantageously, the process of the invention allows to obtain from a keratin-containing material, e.g., pig hair, two products: a liquid fraction and a solid fraction. The liquid fraction of the keratin hydrolysate, comprising: peptides having a molecular weight comprised between 150 Da (Dalton) and 10.000 Da (Dalton) and/or free amino acids.

The solid fraction of the keratin hydrolysate comprises two further fractions: a soluble fraction comprising peptides having a molecular weight comprised between 150 Da (Dalton) and 10.000 Da (Dalton) and a not-soluble (or insoluble) fraction comprising proteins having a molecular weight superior to 10.000 Da (Dalton).

According to embodiments, the liquid mixture of the invention is the liquid fraction of the keratin hydrolysate, the soluble fraction of the solid fraction of the keratin hydrolysate, or a mixture thereof.

According to embodiments, at least the steps of microbial keratin decomposition, the step of steam explosion, the step of enzymatic hydrolysis and the step of treating the keratin hydrolysate to obtain a liquid mixture comprising peptides and/or amino acids are performed consecutively.

According to embodiments, the step of decomposing a keratin containing material in presence of at least one species of microorganism apt to ferment keratin, is a solid state microbial keratin decomposition step. This step consists of depositing a solid culture substrate, in this case pig hair, on flatbeds after seeding it with the consortium of microorganisms; the substrate is then left in a temperature/humidity-controlled room for several days. In this step, an anaerobic area is sought.

According to embodiments, said at least one microorganism apt to decompose keratin is a microorganism, preferably a consortium of microorganisms, which is able to grow using keratin as the sole carbon and nitrogen source.

According to embodiments, said at least one microorganism apt to decompose keratin is a microorganism, preferably a consortium of microorganisms, which excretes hydrolytic enzymes, wherein the hydrolytic enzymes are selected from the group consisting of alkaline proteases such as keratinases which is composed of three type of enzymes: endo-acting, an exo-acting, and an oligopeptide-acting keratinase. According to embodiments, said at least one microorganism is selected from the group consisting of the microorganism deposited under accession number CECT 30381, the microorganism deposited under accession number CECT 30382, the microorganism deposited under accession number CECT 30383, and mixture thereof.

The microorganism deposited under accession number CECT 30381, was deposited on 3^{rd} June 2021 at the International Depositary Authority COLECCIÓN ESPAÑOLA DE CULTIVOS TIPO (CECT), with address Edificio 3 CUE, Parc Científic Universitat de Valencia, Catedrático Agustín Escardino, 9, 46980 Paterna (Valencia) ESPAÑA, by Symborg SLU, with address Avda. Jesus Martinez Cortado, 51, Poligono Industrial Cabezo Cortado, 30100 Espinardo (Murcia), Spain. The identification reference given to this microorganism by the depositor was SB0090.

The microorganism deposited under accession number CECT 30382, was deposited on 3^{rd} June 2021 at the International Depositary Authority COLECCIÓN ESPAÑOLA DE CULTIVOS TIPO (CECT), with address Edificio 3 CUE, Parc Científic Universitat de Valencia, Catedrático Agustín Escardino, 9, 46980 Paterna (Valencia) ESPAÑA, by Symborg SLU, with address Avda. Jesus Martinez Cortado, 51, Poligono Industrial Cabezo Cortado, 30100 Espinardo (Murcia), Spain. The identification reference given to this microorganism by the depositor was SB0091. The microorganism deposited under accession number CECT 30383, was deposited on 3^{rd} June 2021 at the International Depositary Authority COLECCIÓN ESPAÑOLA DE CULTIVOS TIPO (CECT), with address Edificio 3 CUE, Parc Científic Universitat de Valencia, Catedrático Agustín Escardino, 9, 46980 Paterna (Valencia) ESPAÑA, by Symborg SLU, with address Avda. Jesus Martinez Cortado, 51, Poligono Industrial Cabezo Cortado, 30100 Espinardo (Murcia), Spain. The identification reference given to this microorganism by the depositor was SB0092. According to embodiments, the decomposition of a keratin containing material is carried out by, i.e., in presence of, a mixture including at least two, optionally all, of: the microorganism deposited under accession number CECT 30381, the microorganism deposited under accession number CECT 30382 and the microorganism deposited under accession number CECT 30383.

According to embodiments, said at least one microorganism apt to decompose keratin is a bacterium, preferably a bacterium of the *Bacillus* genus.

According to embodiments, the bacterium of the *Bacillus* genus is selected from the group consisting of *Bacillus subtilis, Bacillus Pumilis, Bacillus Cereus, Bacillus licheniformis, Bacillus thermophiles, Bacillus pseudofirmis,* and mixtures thereof. Other microorganisms suitable to be used according to the invention are, for example, keratinolytic bacteria such as *Microbacterium arborescens, Kocuria rosea, Thermoanaerobacter keratinophilus, Xanthomonas maltophila, Fervidobacterium pennavorans, Stenotrophomonas, Chryseobacterium,* keratinolytic fungi *Trichoderma* sp., and mixtures thereof.

According to embodiments, the decomposition of a keratin containing material is carried out by, i.e., in presence of, a consortium of microorganisms selected from two or more of *Bacillus subtilis, Bacillus Pumilis, Bacillus Cereus, Bacillus licheniformis, Bacillus thermophiles, Bacillus pseudofirmis, Microbacterium arborescens, Kocuria rosea, Thermoanaerobacter keratinophilus, Xanthomonas maltophila, Fervidobacterium pennavorans, Stenotrophomonas, Chryseobacterium,* and a keratinolytic fungi a *Trichoderma* sp.

Advantageously, the step of decomposition of a keratin containing material allows for a first breaking of the keratin protein structure.

The decomposed keratin, i.e., the decomposed keratin containing material obtained as above explained, is subsequently treated by steam explosion.

Steam explosion is a process in which biomass (in the present application the decomposed keratin containing material) is treated with hot steam at a temperature from 110 °C to 160°C, under pressure from 1 to 5 bar, that results in a rupture of the keratin fibrous structure.

For example, according to the present invention, the solution of pig hair (20% w/v) is preheated at atmospheric pressure and a temperature of 63 °C.

The pressure is then increased till about 1.3 bar and the temperature reach about 130 °C.

The temperature of the solution of pig hair is cool down till about 55 °C.

Advantageously and according to the present invention, steam explosion of the decomposed keratin containing material obtained according to the above, results in a material, i.e., a modified keratin containing material, which is particularly susceptible to enzymatic hydrolytic modification, i.e., to enzymatic hydrolysis.

According to embodiments, the decomposed keratin containing material is pre-heated, preferably at a temperature from 70° to 90°C, before its mixing with the steam. According to embodiments, once the pig hair is partial degraded or decomposed by the action of microorganisms, said pig hair is milled into small pieces (microns) that are then diluted into water in a proportion of 15%-40% w/v, preferably 20% w/v. According to embodiments, the decomposed keratin containing material is mixed with steam in an amount from 15% to 40%, preferably from 15% to 35% by weight, more preferably from 15% to 30% by weight with respect to the final weight of the mixture. According to embodiments, the decomposed keratin is treated with hot steam at a temperature from 110 °C to 160 °C, preferably from 120 °C to 150 °C.

According to embodiments, the decomposed keratin is treated with hot steam under a pressure from 1 to 5 bar, preferably from 2 to 4 bar.

According to embodiments, the fermented keratin is treated with hot steam under pressure for a time from 2 to 30 min, preferably from 5 to 25 min.

According to an aspect, the step of treating the fermented keratin containing material comprises a step of steam.

According to embodiments, the decomposed keratin containing material is mixed with hot steam by passing hot steam in counter current with respect to the decomposed keratin. Preferably, the mixture is subsequently kept a temperature from 110 °C to 160°C, preferably from 120 °C to 150 °C, under pressure from 1 to 5 bar, preferably from 2 to 4 bar, for a time from 2 to 30 min, preferably from 5 to 25 min.

As above mentioned, steam explosion of the decomposed keratin containing material results in a material, i.e., a modified keratin containing material according to the present invention, which is particularly suitable to be further treated through enzymatic hydrolysis.

According to an aspect, the process of the invention further comprises a step of treating the modified keratin containing material obtained as above indicated, with at least a proteolytic enzyme, to obtain a keratin hydrolysate comprising peptides and/or amino acids.

According to embodiments, the proteolytic enzyme is selected from the group consisting of bacterial, fungal and vegetal, endoproteases and exoproteases preferably the Bacillus alkaline and neutral Bacillus proteases, subtilisin, and mixtures thereof.

According the invention, the step of treating the modified keratin containing material with at least a proteolytic enzyme is carried out using isolated proteolytic enzymes. In other words, according to embodiments, such proteolytic enzymes are isolated from the cell (e.g., a bacterium) in which the enzymes are produced. Enzymes may be isolated and/or purified from cells and organisms according to techniques that are known in the art, obtaining enzymatic preparations.

According to embodiments the substrate, i.e., the modified keratin containing material, concentration is between 10 and 30% weight/volume of the reaction mixture.

According to embodiments, the protease concentration may be between 0.1 and 1.0% weight/volume of reaction mixture, when the enzymatic activity is at least 650 Enzymatic Activity Units/g (gram of enzymatic preparation).

Then, in embodiments, water is added till have a concentration of pig hair of about 15% at a pH value of about 9.0 and a temperature of about 55 °C.

Then, according to embodiments, the enzyme is added in a concentration of 0.15% when the enzymatic activity value is at least 650 Enzymatic Activity Units/g. According to embodiments, the proteolytic enzyme is in an amount from 0.1 to 1.0%, preferably from 0.1 to 0.7%, and the enzymatic activity value is 650 Enzymatic Activity Units/g.

According to embodiments, enzymatic hydrolysis is carried out at a pH from 7 to 10, preferably from 7 to 9.

According to embodiments, enzymatic hydrolysis is carried out at a temperature from 25 °C to 75 °C, preferably from 30 °C to 70 °C.

According to embodiments, enzymatic hydrolysis is carried out for a time in the range from 1 to 24 h, preferably from 2 h to 18 h.

Advantageously, through the enzymatic hydrolysis step according to the present invention, keratin is converted into a hydrolysate containing peptides and/or amino acids.

During enzymatic hydrolysis peptide bonds of keratin are broken, producing peptides and, optionally, free amino acids.

It has been observed that the hydrolysis step according to the invention is particularly efficient, and allows to avoid the destruction or modification of certain amino acid residues. The enzymatic breakdown is mild and does not lead to the destruction or racemisation of amino acids. All amino acids are preserved, in contrast to chemical hydrolysis, which leads to the partial destruction of aromatic and sulphur amino acids, such as tryptophan, cysteine and methionine.

According to an aspect, the process of the invention comprises a step of treating the keratin hydrolysate, obtained after the enzymatic hydrolysis according to the present invention, to obtain a liquid mixture comprising peptides and/or amino acids. According to embodiments, at the end of the enzymatic hydrolysis, the solid fraction and the liquid fraction of the obtained hydrolysate are separated. In other words, according to embodiments, the step of treating said keratin hydrolysate to obtain a liquid mixture comprising peptides and/or amino acids, comprises a step of separating the liquid fraction of said hydrolysate from the solid fraction of the hydrolysate. As above discussed, the solid fraction of the keratin hydrolysate comprises two fractions: a soluble fraction comprising peptides having a molecular weight comprised between 150 Da (Dalton) and 10.000 Da (Dalton), as the liquid fraction, and a not-soluble fraction comprising proteins having a molecular weight superior to 10.000 Da (Dalton).

The solid fraction includes the keratin/protein that wasn't hydrolysed by the enzyme. It also contains some peptides that have not been removed in the separation process, as well as free amino acids.

Solid-liquid separation can be carried out by means of techniques that are known, per se, in the art. For example, the keratin hydrolysate may be treated by centrifugation, or, evaporation concentration to separate the liquid fraction from the solid fraction. In embodiments, the solid fraction may be further treated to separate the soluble fraction of the solid fraction from the insoluble fraction of the solid fraction. Advantageously, the process of the invention allows for the production of a liquid mixture, containing peptides and/or amino acids derived from keratin.

As above discussed, according to embodiments, the liquid mixture of the invention is the liquid fraction of the keratin hydrolysate, the soluble fraction of the solid fraction of the keratin hydrolysate, or a mixture thereof.

Advantageously, the liquid mixture of the invention, e.g., the liquid fraction of the keratin hydrolysate, can be used, according to the invention, as a plant bio-stimulant, suitable for stimulate and/or improve germination, rooting, growth, flowering, curdling and maturation of plants and fruits.

Another object of the present invention is a liquid mixture comprising peptides and/or amino acids as obtainable according to the process of the invention.

According to the embodiments, the liquid mixture comprises from 1% to 10% by weight of free amino acids, from 15% to 30% by weight of peptides having a molecular weight >10000 Da, from 25% to 40% by weight of peptides having a molecular weight of 10000 Da to 400 Da, and 35% to 50% by weight of peptides having a molecular weight <400Da.

Preferably, the liquid mixture comprises from 3% to 6% by weight of amino acids, from 22% to 27% by weight of peptides having a molecular weight > 10000 Da, from 30% to 35% by weight of peptides having a molecular weight from 10.000 Da to 400 Da, and from 40% to 45% by weight of peptides having a molecular weight <400Da. The amount of amino acids and peptides in the liquid mixture, and their molecular weight can be determined, for example, by HPLC (High-performance liquid chromatography), particularly HPLC-DAD (Diode-Array-Detector), or UPLC-UV. As above mentioned, at the end of the enzymatic hydrolysis, the solid fraction and the liquid fraction of the obtained hydrolysate are separated.

In embodiments, the solid fraction of the hydrolysate comprises residual moisture in an amount from 70% to 80% by weight.

Advantageously, the solid fraction of the keratin hydrolysate particularly the not-soluble fraction of the solid fraction of the keratin hydrolysate, as obtainable according to the process of the invention, can be used as fertilizer, according to conventional techniques.

Another object of the invention is the use of the liquid mixture comprising peptides and/or amino acids according to the invention plant bio-stimulant.

According to embodiments, the liquid mixture may be used to improve and/or stimulate one or more of germination, rooting, growth, flowering, curdling and maturation of plants and fruits.

As above discussed, the present invention has several advantages over the known processes for converting keratin into peptides and/or amino acids. In particular, the process of the invention allows to obtain a liquid mixture comprising peptides and/or amino acids suitable to be used as agricultural bio-stimulant, for example, to improve and/or stimulate germination, rooting, growth, flowering, curdling and maturation of plants and fruits. Also, advantageously, the process of the invention allows to obtain a solid mixture, i.e., the solid fraction of the keratin hydrolysate, particularly the insoluble fraction of said solid fraction, that is suitable to be used as fertilizer. The use as fertilizer of the solid fraction of the keratin hydrolysate, particularly of the insoluble fraction of said solid fraction, is another object of the present invention. The present process allows to completely or substantially completely convert the keratin contained in materials such as, for example, pig hair, into compounds, e.g., peptides and/or amino acids, that are more bio-available and that can be used in agriculture as bio-stimulants. The process of the invention allows for the conversion of keratin, thus reducing the amount of keratin-containing materials to be disposed, and that can potentially cause environmental pollution.

These and other advantages of the present invention will be illustrated in the following experimental section, with reference to the following non-limiting examples.

### Experimental section

### Example 1 ― process for obtaining a liquid mixture comprising peptides and/or amino acids from keratin

10000 g of keratin deriving from milled pig hair was processed.

### Step a) Fermentative Phase (Treatment with microorganisms)

A consortium of microorganisms of the *Bacillus* genus, excreting hydrolytic enzymes the most important in terms of hydrolytic potency and amount of expression is subtilisin additionally other expressed enzymes are e Gamma-glutamyltranspeptidases (GGT) and acylaminoacyl.peptidases was used.

Microbial decomposition was carried out in a solid state.

Keratin with a humidity between 40-60% was introduced into a solid state bioreactor and was inoculated with the bacterial consortium.

The inoculum is in an amount of 1-10% by weight with respect to the weight of the keratin material, and has a bacterial concentration that is about 10⁹ CFU/g.

The solid state bioreactor has the following characteristics. The height of the pile will be between 20 to 60 centimeters. The width of the pile from 60 to 120 cm, and the length has no limit.

Keratin was kept between 25°C and 45°C, preferably between 30°C and 50°C, at a humidity between 40-60%. The microbial keratin decomposition time is between 15 and 30 days.

During this step, keratin is modified due to the high amount of hydrolytic enzymes excreted by the microorganism during the fermentation.

The fermenting step is controlled by measuring keratin protein solublization for example by difference of mass. A known amount of pig hair is put into the bioreactor, and when the insoluble matter loss a 30% of mass the process is considered ended. When the keratin solubilization reaches 30%, the microbial keratin decomposition phase is ended.

### Step b) Steam explosion

The decomposed partially soluble keratin is treated in a continuous process, where the mixture reached a pressure of 2 bar and at a temperature of 120°C, and is kept at this pressure and temperature for 20 minutes. The mixture reduces its temperature to 50-60 °C, preferably 45°C to 55°C, thus obtaining a sterile product ready to be used in the hydrolytic phase.

This steam explosion treatment leads to a solubilization of the proteins, and modification of keratin protein structure.

The solubilization of the protein material is evident, and this process leads to a mixture containing products that are more-bioavailable with respect to keratin.

### Step c) enzymatic hydrolysis

The pH of mixture obtained after steam explosion was adjusted to a value of 9.0 with 10 M calcium hydroxide.

The mixture was treated at 55°C in a batch bioreactor under stirring (60-80 rpm) with 0.1% by volume of subtilisin having an enzymatic activity that is at least 650 enzymatic activity units/g. The pH was maintained with a chemical base as calcium hydroxide, Ca(OH)2, around 9.5. Hydrolysis can be continued for 2-24 hours.

After hydrolysis was completed, the hydrolysate was processed in several stages: centrifugation (using a decanter or a disc centrifuge), filtration and concentration in an evaporator.

The liquid fraction and the solid fraction of the keratin hydrolysate are, therefore, separated.

Characteristics of such products, as well as characteristics of keratin before the process are reported in Table 1.

The amino acids composition of the liquid fraction of the hydrolysate, of the solid fraction of the hydrolysate and of the keratin before the process is reported in Table 2.

Percentages are indicated as weight percentages.

| **Table 1** | | | |
|---|---|---|---|
| | Pig hair (Keratin source) | Liquid fraction of the hydrolysate | Solid fraction of the hydrolysate |
| Humidity % (w/w) (Desiccation at 105 °C) | 12,1 | 88,20 | 70.9 |
| Dry Matter % (w/w) (Desiccation at 105 °C) | 87,9 | 11,80 | 29.1 |
| ensity to 20°C (g/cc) (Gravimetry) | | 1.047 g/cc | |
| pH | 4,3 | 9,81 | 9,8 |
| Ash % (w/w) Calcination at 800 °C according to the UNE-EN 13039 | | 1,6 | |
| Total Organic Matter % (w/w) (calcination according to the UNE-EN 13039) | | 10,21% | |
| Total organic carbon % (w/w) | 56 | 50,10 | |
| Total nitrogen % (w/w) (Dumas method, based on UNE-EN 13654-2 | | 1,8 | |
| Ammonia nitrogen % (w/w) (Ion chromatography, based on UNE-EN 14911) | | 0.45 | |
| Nitric nitrogen (mg/kg) (Ion chromatography, based on UNE-EN 10304-1) | | < 22.6 | |
| Ureic nitrogen % (w/w) (HPLC-UV, based on UNE-EN ISO 19746) | | < 0.1 | |
| Organic nitrogen % (w/w) (Math calculation, based on the Spanish regulation R.D. 1110/1991 annex Num. 4) | | 1.4 | |

| **Table 2** | | | |
|---|---|---|---|
| Amino acids (weight %) | Keratin | Liquid fraction of the hydrolysate | Solid fraction of the hydrolysate |
| Aspartic acid | 5,3 | 0,655 | 1,43 |
| Gamma-aminobutyric acid | 0,064 | < 0,00500 | 0,0136 |
| Glutamic acid | 11,52 | 1,952 | 3,71 |
| Alanine | 3,07 | 0,411 | 0,87 |
| Arginine | 5,3 | 0,649 | 1,72 |
| Phenylalanine | 1,56 | 0,183 | 0,5 |
| Glycine | 3 | 0,425 | 0,86 |
| Hydroxyproline | < 0,00439 | < 0,00500 | < 0,00146 |
| Histidine | 0,5 | 0,046 | 0,15 |
| Isoleucine | 2,1 | 0,281 | 0,65 |
| Leucine | 5,21 | 0,653 | 1,6 |
| Lysine | 2,47 | 0,298 | 0,64 |
| Methionine | 0,54 | 0,0749 | 0,16 |
| Proline | 3,96 | 0,487 | 1,25 |
| Serine | 4,27 | 0,531 | 1,31 |
| Tyrosine | 2,2 | 0,28 | 0,72 |
| Threonine | 2,99 | 0,321 | 0,9 |
| Tryptophan | 0,329 | 0,0415 | 0,154 |
| Valine | 3,76 | 0,498 | 1,15 |
| Sum of total amino acids | 58,1 | 7,78 | 17,8 |

Method: hydrolysis and UPLC-UV, AccQTag Ultra Derivation Kit

The total amino acids content is by math calculation.

The mass yield of obtaining the liquid fraction of the hydrolysate is in the range from 55% to 70%, while the solid faction of the hydrolysate represents the rest of the treated keratin.

Advantageously, the process of the invention does not produce or substantially produce waste materials.

### Example 2 ― analysis of a mixture containing peptides and/or amino acids obtained through the process of the invention

An exemplary mixture containing peptides and/or amino acids according to the invention was analyzed.

The mixture is the liquid fraction of a keratin hydrolysate obtained through the process of the invention.

Data in Table 3 are expressed as weight percentage with respect to the dry weight of the liquid mixture.

| **Table 3** | |
|---|---|
| Total organic carbon | 47,68% |
| Total nitrogen | 16,45% |
| Organic nitrogen | 16,45% |
| N-NH₄ | n.d. |
| Free amino acids | 8,51% |

The mixture containing peptides and/or amino acids according to the invention was also analyzed through HPLC in order to determine the distribution of different kind of compounds (i.e., peptides having different molecular weight and/or aminoacids) in the mixture. The results are reported in Table 4.

| **Table 4** | |
|---|---|
| Compounds having molecular weight <400 Da | 49.6% |
| Compounds having molecular weight from 400 Da to 10000 Da | 32.9% |
| Compounds having molecular weight >10000 Da | 17.4% |

## Claims

1. A process for converting keratin into a liquid mixture comprising peptides and/or amino acids comprising the following steps:
a) decomposing a keratin containing material, preferably pig hair, in presence of at least one species of microorganism apt to decompose keratin, to obtain a decomposed keratin containing material;
b) treating the decomposed keratin containing material to obtain a modified keratin containing material;
c) treating said modified keratin containing material with at least a proteolytic enzyme to obtain a keratin hydrolysate, said keratin hydrolysate comprising a liquid fraction and a solid fraction, said solid fraction comprising two further fractions: a soluble fraction and a not-soluble fraction; and
d) treating said keratin hydrolysate to obtain a liquid mixture comprising peptides and/or amino acids.

2. Process according to claim 1, wherein said at least one species of microorganism apt to decompose keratin is a consortium of microorganisms able to grow using keratin as the sole carbon and nitrogen source.

3. Process according to claim 1, wherein said at least one microorganism is selected from the group consisting of the microorganism deposited under accession number CECT 30381, the microorganism deposited under accession number CECT 30382, the microorganism deposited under accession number CECT 30383, and mixture thereof.

4. Process according to claim 1, wherein said at least one species of microorganism apt to decompose keratin is selected from the group consisting of *Bacillus subtilis, Bacillus Pumilis, Bacillus Cereus, Bacillus licheniformis, Bacillus thermophiles, Bacillus pseudofirmis, Microbacterium arborescens, Kocuria rosea, Thermoanaerobacter keratinophilus, Xanthomonas maltophila, Fervidobacterium pennavorans, Stenotrophomonas, Chryseobacterium, Trichoderma sp.,* and mixtures thereof.

5. Process according to claim 1, wherein, in said step of treating said modified keratin containing material with at least a proteolytic enzyme, said proteolytic enzyme selected from the group consisting of subtilisin, microbial, fungal and vegetal, endoproteases and exoproteases preferably the Bacillus alkaline and neutral Bacillus proteases and mixtures thereof.

6. Process according to claim 1, wherein said step d) of treating said keratin hydrolysate comprises a step of separating said liquid fraction from said solid fraction.

7. A liquid mixture comprising peptides and/or amino acids as obtainable through to the process according to claim 1.

8. Liquid mixture according to claim 7, wherein said liquid mixture is the liquid fraction of said hydrolysate obtained after said step of treating said modified keratin containing material with at least a proteolytic enzyme, or the soluble fraction of the solid fraction of the hydrolysate obtained after said step of treating said modified keratin containing material with at least a proteolytic enzyme, or a mixture thereof.

9. Use of the liquid mixture according to claim 7 as a bio-stimulant of plants.

10. Use of the liquid mixture according to claim 7 in cosmetic field or animal nutrition.

11. Use according to claim 9, to stimulate one or more of germination, rooting, growth, flowering, curdling and maturation of plants and fruits.

12. Use of said not-soluble fraction according to claim 1, as fertilizer.

13. Microorganism deposited under accession number CECT 30381.

14. Microorganism deposited under accession number CECT 30382.

15. Microorganism deposited under accession number CECT 30383.
